# EUROPÄISCHE PATENTSCHRIFT

(11) **EP 3 687 685 B1**
(45) Veröffentlichungstag und Bekanntmachung des Hinweises auf die Patenterteilung: **28.10.2026**
(21) Anmeldenummer: 18719791.8
(22) Anmeldetag: 13.04.2018
(51) Int. Cl.: C12Q 1/6806, B01L 3/00, C12N 15/10, B03C 1/01, B03C 1/033, B03C 1/28, G01N 33/543, G01N 35/00

(54) **VORRICHTUNG UND VERFAHREN ZUR IMMOBILISIERUNG VON BIOMOLEKÜLEN MIT MAGNETISCHEN PARTIKELN**
DEVICE AND METHOD FOR IMMOBILISING BIOMOLECULES USING MAGNETIC PARTICLES
DISPOSITIF ET PROCÉDÉ POUR IMMOBILISER DES BIOMOLÉCULES AU MOYEN DE PARTICULES MAGNÉTIQUES

(30) Priorität: 25.09.2017 US 201762562647 P
(43) Veröffentlichungstag der Anmeldung: 05.08.2020
(73) Patentinhaber: QIAGEN GmbH, 40724 Hilden (DE)
(72) Erfinder: TIEDTKE, Hans-Jürgen, 53227 Bonn (DE); QUINTEL, Harald, 8266 Steckborn (CH); LUTZE, Konstantin, 8713 Uerikon (CH)
(74) Vertreter: IPS Irsch AG
(86) Internationale Anmeldenummer: PCT/EP2018/059543
(87) Internationale Veröffentlichungsnummer: WO 2019/057345

(56) Entgegenhaltungen:
- WO-A1-2016/061285
- WO-A1-92/05443
- DE-A1- 19 834 584
- PENG HAN ET AL: "Design and Fabrication of a Hybrid HTS Magnet for 150 kJ SMES", JOURNAL OF FUSION ENERGY, 1 December 2014 (2014-12-01), Boston, pages 759 - 764, XP055484758, Retrieved from the Internet <URL:http://www2.lbl.gov/Tech-Transfer/publications/1714LBNL_Report.pdf> DOI: 10.1007/s10894-014-9744-z
- XAVIER LE GUEZENNEC ET AL: "Improving Bead-Based Multiplexing in Bodily Fluids", GENETIC ENGINEERING & BIOTECHNOLOGY NEWS, vol. 36, no. 2, 15 January 2016 (2016-01-15), pages 1 - 4, XP055484818

## Beschreibung

Die Erfindung betrifft eine Vorrichtung zur reversiblen Immobilisierung von Biomolekülen gemäss dem Oberbegriff des unabhängigen Anspruchs 1. Die Erfindung betrifft weiter ein Verfahren zur reversiblen Immobilisierung von Biomolekülen gemäss dem Oberbegriff des unabhängigen Anspruchs 12 Die Erfindung betrifft weiter einen Apparat zur automatisierten Verarbeitung von Biomolekülen umfassend eine Vorrichtung zur reversiblen Immobilisierung von Biomolekülen gemäss dem Oberbegriff des unabhängigen Anspruchs 13

Die Erfindung wird durch die Ansprüche definiert. Jeglicher Gegenstand der außerhalb des Bereichs der Ansprüche liegt ist lediglich zu Informationszwecken beschrieben. Im Stand der Technik sind viele Methoden zur Aufreinigung von DNS und anderen Biomolekülen bekannt. Eine Art der Aufreinigung ist die DNS-Extraktion, bei welcher die DNS in unpolarer Umgebung ausgefällt wird. Auch kann DNS über Zentrifugation, z.B. nach einem Zellaufschluss gereinigt werden oder über elektrophoretische Methoden.

Biomoleküle können auch durch Immobilisierung auf einem unlöslichen Träger synthetisiert und gereinigt werden. Übliche Substrate um Biomoleküle zu immobilisieren sind Glas sowie anderen, weniger verbreitete Substrate wie Gold, Platin, Oxide, Halbleiter und diverse Polymersubstrate.

Da eine manuelle Aufreinigung und Verarbeitung bei zahlreichen Arbeitsgängen zu viel Zeit erfordert erfolgen die Prozesse heutzutage vollautomatisiert. So spielt für die Automatisierung von Labormethoden sogenannte "magnetic beats" (magnetische Partikel) eine grosse Rolle.

"Magnetic bead-based clean-up" und "magnetic bead-based normalization" sind weit verbreitete Verfahren zur Immobilisierung, Aufreinigung und Konzentrationsanpassung von Nukleinsäuren. Typische Anwendungsgebiete dieser Verfahren sind die Probenvorbereitung im Kontext von DNS Sequenzierung oder DNS Detektion (z.B. mittels PCR, englisch polymerase chain reaction, deutsch Polymerase-Kettenreaktion).

Im Stand der Technik werden die magnetischen Partikel typischerweise durch Ringmagnete, welche einen Behälter umschliessen, im Behälter gehalten. Dadurch kann eine Lösung mit Verunreinigungen abpipettiert werden, während die magnetischen Partikel mit den gebundenen Biomolekülen im Behälter verbleiben.

Die magnetischen Partikel (magnetic beads) wurden 1995 am Whitehead Institute zu Aufreinigung von PCR Produkten entwickelt. Die magnetischen Partikel sind meist paramagnetisch und können z.B. aus Polystyrol bestehen, welches mit Eisen beschichtet ist. Auf dem Eisen können dann verschiedene Moleküle mit Carboxylgruppen angebracht sein. Diese Carboxylgruppen können die DNS-Moleküle reversibel binden. Dadurch werden die DNS-Moleküle immobilisiert.

Verfahren mit magnetischen Partikeln umfassen meist folgende Schritte. Zuerst werden die PCR-Produkte an die magnetischen Partikel gebunden. Anschliessend werden die magnetischen Partikel mit den angehefteten PCR-Produkten von Verunreinigungen getrennt (dieser Schritt wird z.B. durch abpipettieren der Lösung vom Feststoff realisiert). Dann werden die magnetischen Partikel mit den angehefteten PCR-Produkten gewaschen. Nach dem Waschen werden die PCR-Produkte von den magnetischen Partikeln eluiert und auf eine neue Platte transferiert.

Bei vollautomatisierten Prozessen werden nach dem Einbringen des Ausgangsmaterials in einem Isolationsprozess die notwendigen Reagenzien automatisch zur Probe pipettiert und mittels Pipettenspitze wieder entfernt. Die Magnetpartikel-gebundenen Nukleinsäuren werden am Boden und am Rand der Kavitäten gesammelt und in Abhängigkeit der Routine durch optimiertes Auf- und Abpipettieren wiederum in Lösung gebracht. Final werden die DNS oder die RNS für eine direkte Lagerung oder weitere Anwendungen in separate Gefäße mit Deckel eluiert.

Auch sind im Stand der Technik Adsorptionsmethoden bekannt, bei welchen die DNS z.B. in leicht saurer Umgebung an Silica-Gel gebunden wird.

So ist eines der wichtigsten Verfahren zur Synthese, Normalisierung und zum Reinigen von Biomolekülen das Verfahren mit magnetischen Partikeln. Hierbei werden die Biomoleküle an die Oberfläche von den magnetischen Partikeln gebunden. Die magnetischen Partikel werden dann mittels eines Magneten fixiert und die Lösung, in welcher sich Nebenprodukte und Verunreinigungen befinden, können einfach abgetrennt werden. So können die Biomoleküle einfach und schnell gereinigt und isoliert werden. Der Vorteil der magnetischen Kügelchen liegt darin, dass sich die Kügelchen im Versuchsansatz frei bewegen können, was wichtig für die Bindungsschritte ist. Will man nun z.B. in einem Waschschritt die Flüssigkeit aus dem Gefäss entfernen, hält man einfach einen Magneten an den Behälter und kann dann die Flüssigkeit abtrennen.

Bei den magnetischen Partikeln (auch magnetic beads genannt) handelt es sich um kleine para- oder ferromagnetische Kügelchen, welche mit verschiedenen Materialien beschichtet werden, welche die benötigten Eigenschaften vermitteln. Oft werden Nickelpartikel verwendet, welche mit einem Kunststoff beschichtet sind.

So können z.B. auch DNS-Sonden und Gene in automatisierten Festphasenmethoden synthetisiert werden. DNS-Stränge kann man, genau wie Polypeptide durch das aufeinanderfolgende Anhängen aktivierter Monomere an eine wachsende Kette, die an eine unlösliche Matrix (magnetische Partikel) gebunden ist, synthetisieren. Als aktivierte Monomere können hier geschützte Phosphoramidite dienen.

Dieses Vorgehen erlaubt die Isolierung hochreiner Biomoleküle mit exzellenten Ausbeuten. Das zugrundeliegende Verfahren der Magnetpartikelseparation kann dabei vollautomatisiert in den Kavitäten von verwendeten Extraktionsbehältern erfolgen.

Bei vollautomatisierten Prozessen werden nach dem Einbringen des Ausgangsmaterials in den Isolationsprozess die notwendigen Reagenzien automatisch zur Probe pipettiert und zum Beispiel mittels Pipetten wieder entfernt. Die Magnetpartikel-gebundenen Nukleinsäuren werden am Boden und am Rand der Kavitäten gesammelt und in Abhängigkeit der Routine durch optimiertes Auf- und Abpipettieren wiederum in Lösung gebracht. Final werden die DNS oder die RNS für eine direkte Lagerung oder weitere Anwendungen in separate Gefäße mit Deckel eluiert.

Im Stand der Technik werden die magnetischen Partikel typischerweise durch Ringmagnete, welche den Behälter umschliessen, im Behälter gehalten. Dadurch ordnen sich die magnetischen Partikel im inneren Behälter ringförmig an. Entsprechende Vorrichtungen und Verfahren des Stands der Technik werden in Peng Han et al. (Journal of Fusion Energy, 1. Dezember 2014 , Seiten 759-764), Xavier Le Guezennec (GENETIC ENGINEERING&BIOTECHNOLOGY NEWS, Bd. 36, Nr. 2, 15. Januar 2016, Seiten 1-4), WO 2016/061285 (ALPAQUA ENGINEERING LLC [US]), und WO92/05443 (MEDICAL RES COUNCIL [GB]) beschrieben.

Ein wesentlicher Nachteil des Standes der Technik ist, dass sich durch die Verwendung von Ringmagneten auch die magnetische Partikel im Behälter in einem Ring anordnen. Dadurch lässt sich nicht nur die Flüssigkeit schwerer entfernen, sondern es bleibt auch immer noch ein nicht zu entfernender Flüssigkeitsrest auf dem Feststoffring erhalten. Die unvollständige Entfernung der Flüssigkeit mindert die Reinigungseffizienz und reduziert das nutzbare Volumen nach der Elution.

Aufgabe der Erfindung ist es daher eine Vorrichtung zur Immobilisierung von Biomolekülen, ein Verfahren zur reversiblen Immobilisierung von Biomolekülen und einen Apparat zur automatisierten Verarbeitung von Biomolekülen mit einer Vorrichtung zur Immobilisierung von Biomolekülen bereitzustellen, welche die aus dem Stand der Technik bekannten nachteiligen Wirkungen vermeiden.

Die Aufgabe wird durch eine Vorrichtung zur reversiblen Immobilisierung von Biomolekülen mit den Merkmalen des unabhängigen Anspruchs 1, durch ein Verfahren zur reversiblen Immobilisierung von Biomolekülen mit den Merkmalen des unabhängigen Anspruchs 12 und durch einen Apparat zur automatisierten Verarbeitung von Biomolekülen umfassend eine Vorrichtung zur reversiblen Immobilisierung mit den Merkmalen des unabhängigen Anspruchs 13 gelöst.

Erfindungsgemäss wird eine Vorrichtung zur reversiblen Immobilisierung von Biomolekülen mittels magnetischer Partikel vorgeschlagen. Diese Vorrichtung umfasst einen mit einer Flüssigkeit mit Biomolekülen befüllbaren Behälter und einen Magnet. Der Magnet ist derart am Behälter angeordnet, dass im Behälter anordnenbare magnetische Partikel an welchen die Biomoleküle immobilisierbar sind, insbesondere reversibel immobilisierbar sind, im Behälter fixierbar sind. Durch Anordnung des Magneten ist ein, auf die im Behälter befindlichen magnetischen Partikel wirkendes, inhomogenes Magnetfeld erzeugbar. Die magnetischen Partikel sind durch Beeinflussung von dem inhomogenen Magnetfeld strukturiert anordnenbar.

Wesentlich für die Erfindung ist, dass durch die Anordnung des Magneten Inhomogenität im Magnetfeld erzeugt wird. Folglich wird durch die Anordnung des Magneten ein inhomogenes Magnetfeld erzeugt, welches auf die im Behälter angeordneten magnetischen Partikel wirkt, sodass durch strukturierte Anordnung der magnetischen Partikel im inhomogenen Magnetfeld, die Flüssigkeit leichter von den magnetischen Partikeln abfliessen kann. Wenn sich die magnetischen Partikel also, gesteuert durch das inhomogene Magnetfeld, in isolierten Inseln an einer Behälterwand anordnen, kann die Flüssigkeit einfach zwischen den magnetischen Partikeln abfliessen und somit die Flüssigkeit leichter aus dem Behälter entfernt werden.

Im Rahmen der vorliegenden Erfindung können unter der Anordnung des Magneten verschiedenste Ausführungen verstanden werden. Die Anordnung der Magneten bezieht sich auf die Funktion ein derart inhomogenes Magnetfeld zu erzeugen, welches auf die magnetischen Partikel im Behälter einwirkt, sodass sich die magnetischen Partikel in einer erfindungsgemässen Struktur an der Behälterwand anordnen. Durch das Umleiten einer magnetischen Flussdichte des Magneten ordnen sich die magnetischen Partikel im Behälter so an, dass die Flüssigkeit leichter abfliessen kann und leichter entfernbar ist. Unter "erfindungsgemässer Struktur" ist hierbei eine Struktur zu verstehen, welche ein leichteres abfliessen der Flüssigkeit von den magnetischen Partikeln im Behälter ermöglicht. Somit kann unter der "erfindungsgemässen Struktur" beziehungsweise unter "strukturiert anordnen" zum Beispiel die Anordnung der magnetischen Partikel in isolierten Inseln verstanden werden, jedoch ist die Anordnung nicht darauf beschränkt. Die magnetischen Partikel können sich zum Beispiel auch pyramidal oder rillenförmig anordnen. Alle vorrangehend beschriebenen strukturierten Anordnungen der magnetischen Partikel an der Behälterwand erlauben es somit, dass die Flüssigkeit einfach an oder zwischen den magnetischen Partikeln ablaufen kann. Um Beispiele für die Ausführung der Anordnung des Magneten seien im folgenden verschiedene Möglichkeiten zur Anordnung des Magneten. Unter Anordnung des Magneten kann im Rahmen der Erfindung eine besondere Form des Magneten verstanden werden, wobei die Form sich auf die äussere Gestaltung eines Permanentmagneten oder auf die Wicklung einer Spule bei einem Elektromagnet beziehen kann. Des Weiteren kann unter der Anordnung des Magneten ein magnetisch Leitfähiges Modul verstanden werden, durch welches die magnetische Flussdichte verändert wird und somit ein inhomogenes Magnetfeld erzeugt wird. Unter der Anordnung des Magneten kann auch die Anordnung mehrerer Magnete um den Behälter in vorgebbarem Abstand (Abstand vom Behälter und Abstand der Magnete zueinander) verstanden werden, sodass ein resultierendes Magnetfeld in Form des inhomogenen Magnetfeldes auf die magnetischen Partikel im Behälter einwirkt. Hierbei wirkt das resultierende inhomogene Magnetfeld in manchen Bereichen stärker auf die magnetischen Partikel und in anderen Bereichen schwächer auf die magnetischen Partikel ein. Denselben Effekt gibt es selbstverständlich bei den anderen Anordnungen, da auch durch das magnetisch leitfähige Modul und durch Form des Magneten, das Magnetfeld in manchen Bereichen des Behälters stärker auf die im Behälter befindlichen magnetischen Partikel einwirkt. Diese Möglichkeiten werden in der folgenden Beschreibung, sowie der Figurenbeschreibung genauer erläutert. Es sei noch einmal angemerkt, dass unter strukturierter Anordnung keine ringförmigen oder ähnlichen, aus dem Stand der Technik bekannte Anordnungen der magnetischen Partikel, gemeint sind.

Der Magnet kann ausserdem derart beweglich am Behälter angeordnet sein, dass die magnetischen Partikel während einem Reaktionsschritt im Behälter frei bewegbar sind und während eines Waschschritts, durch ändern der Magnetposition im Behälter fixiert werden. Insbesondere kann der Magnet derart bewegbar sein, dass der Magnet in einer ersten Position am Behälter angeordnet ist und die magnetischen Partikel fixiert und durch Bewegung des Magneten in eine zweite Position am oder um den Behälter, die magnetischen Partikel bewegbar werden. Selbstverständlich kann z.B. in einer automatisierten Vorrichtung auch der Behälter relativ zum Magneten bewegt werden und den gleichen Effekt zu erzielen.

Im Rahmen dieser Erfindung ist unter dem Begriff Biomolekül, DNS, RNS, Nukleinsäuren, Proteine, Startsequenzen für Biomoleküle, Monomere oder andere biologisch aktive Moleküle zu verstehen.

Ein Waschschritt ist im Folgenden im Allgemeinen ein Verfahrensschritt, bei welchem die Flüssigkeit aus den Behältern durch Betätigung des Ventils abgelassen wird und bei welchem so die Verunreinigungen von magnetischen Partikeln mit den angehafteten Biomolekülen abgetrennt werden. Zu einem Waschschritt kann auch Waschen mit einer Waschlösung gehören (Wasser oder andere).

Ein Reaktionsschritt ist im Folgenden im Allgemeinen ein Verfahrensschritt, bei welchem die an die Magnetische Partikel gebundenen Biomoleküle umgesetzt, an die Partikel gebunden, oder verlängert (Kettenverlängerung, z.B. PCR "Polymerase-Kettenreaktion") werden.

Eine Verunreinigung ist im Folgenden im Allgemeinen ein Stoff welcher nicht vollständig reagiert ist beziehungsweise nicht an die magnetischen Partikel gebunden ist, das Lösungsmittel, Nebenprodukte und Kontaminationen, sowie ein Gemisch von zwei oder mehrerer der vorrangehend beschriebenen.

Eine Flüssigkeit kann im Rahmen der Erfindung eine Lösung sein, insbesondere ein Reaktionsgemisch aus Biomolekülen und/oder Reagenzien und/oder Verunreinigungen sein.

Ein magnetischer Partikel (auch "magnetic bead") kann im Folgenden im Allgemeinen ein Partikel im Mikrometer oder im Millimeterbereich sein. Weiter kann ein magnetischer Partikel porös sein. Ein Biomolekül kann im Folgenden im Allgemeinen über Thiolgruppen und/oder Aminogruppen und/oder Hydroxygruppen und/oder Carboxylgruppen und/oder Carbonylgruppen und/oder Estergruppen und/oder Nitrilgruppen und/oder Amingruppen und/oder irgendwelchen anderen funktionellen Gruppen an die Oberfläche der magnetischen Partikel gebunden sein. Ein magnetischer Partikel kann ausserdem ein beschichteter Nickelpartikel sein oder irgendein anderer ferro- oder paramagnetischer Partikel. Magnetische Partikel haben typischerweise einen, Durchmesser von ungefähr 1 Mikrometer. Unter ungefähr 1 Mikrometer ist im Rahmen der Erfindung 0,5 bis 1,5 Mikrometer, insbesondere 0,7 bis 1,3 Mikrometer im speziellen 0,9 bis 1,1 Mikrometer zu verstehen.

Die Vorteile der erfindungsgemässen Vorrichtung und des erfindungsgeässen Verfahrens sind:
- geringe Prozesszeiten durch schnelleres Ablaufen.
- Hohe Ausbeuten
- reinere Produkte
- effizient und kostengünstig
- leicht automatisierbar
- auch für Vorrichtungen reduzierter Grösse
- erlaubt einfache Modifizierung vorhandener Maschinen
- Separation kann geschaltet werden ohne das Disposable zu bewegen (nur mit Elektromagnet)

In der Praxis können die Vorrichtung und das Verfahren für Post-Ligation-Aufreinigung verwendet werden.

Der Magnet der erfindungsgemässen Vorrichtung kann als ein Permanentmagnet und/oder ein Elektromagnet ausgestaltet sein. Während bei einem Permanentmagneten die Form Einfluss auf die Homogenität des Magnetfeldes nimmt kann die Homogenität eines Elektromagneten über die Wicklung bestimmt werden. So kann die Form des Permanentmagneten dafür sorgen, dass das Magnetfeld derart inhomogen ist, das sich die magnetischen Partikel durch dessen Beeinflussung strukturiert anordnen. Das strukturiert anordnen kann sich zum Beispiel auf eine Anordnung in mehreren räumlich voneinander getrennten Inseln beziehen, von welchen Flüssigkeit gut abfliessen kann. Bei Verwendung eines Elektromagneten hingegen kann durch eine stellenweise dichtere Wicklung, ein stellenweise stärkeres Magnetfeld erzeugt werden, sodass auch ein inhomogenes Magnetfeld erzeugt wird, in welchem sich die magnetischen Partikel strukturiert anordnen.

Die Anordnung des Magneten kann auch derart ausgeführt sein, dass der Magnet ein magnetisch leitfähiges Modul umfasst, sodass durch das magnetisch leitfähige Modul ein, auf die im Behälter befindlichen magnetischen Partikel wirkendes, inhomogenes Magnetfeld erzeugt wird. Das magnetisch leitfähige Modul muss Feldlinien eines Magnetfeldes des Magneten also derart beeinflussen, dass sich die magnetische Partikel in einer erfindungsgemässen Struktur anordnen. Hierfür kann das Magnetfeld des Magneten durch das magnetisch leitfähige Modul an vorgebbaren Stellen des Behälter verstärkt oder abgeschwächt werden, sodass sich die magnetischen Partikel in den verstärkten Bereichen vermehrt beziehungsweise in den abgeschwächten Bereichen des Behälters vermindert anordnen. So können abwechselnde Teilbereiche im Behälter mit verstärktem oder abgeschwächtem Magnetfeld erzeugt werden. Auch ist es denkbar, dass das magnetisch leitfähige Modul das Magnetfeld des Magneten an vorgebbaren Bereichen verstärkt und an den nicht verstärkten Bereichen abschwächt. Auch die Form des Magneten kann derart angepasst werden, dass das Magnetfeld an vorgebbaren Bereichen des Behälters verstärkt und an den nicht verstärkten Bereichen abschwächt wird.

Ausserdem kann das magnetisch leitfähige Modul als ein Bauteil am Magneten angeordnet sein oder das magnetisch leitfähige Modul kann als ein integriertes Element des Magneten ausgeführt sein. Das magnetisch leitfähige Modul kann also ein Aufsatz für den Magnet sein und ausserdem ein Element, welches zwischen Behälter und Magnet angeordnet ist. Bei eine Ringmagneten zum Beispiel kann ein magnetisch leitfähiges Modul als kleinerer Ring, mit Einbuchtungen oder anderen Umformungen, zwischen Behälter und Magneten angeordnet sein.

Generell kann das magnetisch leitfähige Modul direkt am Magneten angeordnet sein oder in einem vorgebbaren Abstand zum Magneten angeordnet sein. Durch das magnetisch leitfähige Modul wird das homogene Magnetfeld des Magneten zu einem inhomogenen Magnetfeld.

Das magnetisch leitfähige Modul kann als ein magnetisch verstärkendes Modul und/oder ein diamagnetisches Modul ausgeführt sein. Durch das magnetisch verstärkende Modul wird das Magnetfeld des Magneten in vorgebbaren Bereichen des Behälters verstärkt und durch das diamagnetische Modul wird das Magnetfeld des Magneten in vorgebbaren Bereichen des Behälters abgeschwächt. Das diamagnetische Modul könnte auch als mehrere diamagnetische Schilder ausgestaltet sein, welche so am Magneten angeordnet sind, dass das Magnetfeld des Magneten im Behälter stellenweise abgeschirmt und somit abgeschwächt wird.

Das diamagnetische Modul besteht dabei aus einem diamagnetischen Stoff, wie zum Beispiel Graphit, mit einer relativen Permeabilität <1.

Das magnetisch verstärkende Modul besteht aus einen ferro- und/oder paramagnetischen Stoff mit einer relativen Permeabilität >1. Typische ferromagnetische Stoffe sind zum Beispiel Eisen, Nickel und Kobalt. Typische paramagnetische Stoffe sind zum Beispiel Erdalkalimetalle.

In Ausgestaltung der Erfindung kann auch eine Mischung eines diamagnetischen Moduls mit einem magnetisch verstärkendem Modul verwendet werden, indem das Modul verschiedene diamagnetische und ferro-/paramagnetische Teilbereiche aufweist.

In der Praxis kann eine Form des Magneten an eine Form des Behälters angepasst sein, sodass der Behälter in das magnetisch leitfähige Modul einführt werden kann. Selbstverständlich kann somit auch die Form des magnetisch leitfähigen Moduls an den Behälter angepasst sein. Der Magnet umfasst ein Loch zum Einstecken des Behälters. Auch das magnetisch leitfähige Modul kann ein Loch und/oder eine Ausbuchtung zum Einstecken des Behälters umfassen.

Der Behälter kann auf beliebige Art ausgeformt sein. In Ausgestaltung der Erfindung kann der Behälter eine Multiwellplatte sein, wobei die Multiwellplatte mehrere Vertiefungen aufweist. Eine Multiwellplatte kann insbesondere auch eine Mikrotitrierplatte sein. Besonders vorteilhaft kann der Behälter auch eine Kapillare umfassen, in welchem die Flüssigkeit mit den magnetischen Partikeln durch Kapillarkräfte gehalten wird und/oder die Flüssigkeit durch Druck entfernt wird.

An mehreren Vertiefungen der Multiwellplatte kann ein Magnet angeordnet sein. So können in mehreren Vertiefungen gleichzeitig die magnetischen Partikel fixiert werden.

Die Anordnung des Magneten kann auch derart ausgeführt sein, dass ein zweiter Magnet am Magneten derart angeordnet ist, dass das erste Magnetfeld des Magneten durch das zweite Magnetfeld des zweiten Magneten beeinflussbar ist, sodass ein, auf die im Behälter befindlichen magnetischen Partikel wirkendes, inhomogenes Magnetfeld erzeugt wird. Werden als zweiter Magnet mehrere Permanentmagnete verwendet, können die Permanentmagnete so am Magneten angeordnet werden, dass das Magnetfeld des Magneten an manchen Stellen abgeschwächt und/oder verstärkt wird. Auch könnte als zweiter Magnet ein Elektromagnet verwendet werden, der mit seinem zweiten Magnetfeld das Magnetfeld des Magneten in gewünschter Art inhomogenisiert.

In Ausgestaltung der Erfindung kann die Anordnung des Magneten auch derart ausgeführt sein, dass der Magnet eine oder mehrere Einkerbung aufweist, sodass durch die Einkerbung des Magneten ein inhomogenes Magnetfeld erzeugt wird. An den Stellen der Einkerbungen des Magneten wird im Behälter ein schwächeres Magnetfeld erzeugt, sodass sich an den Stellen der Einkerbungen weniger oder keine magnetischen Partikel sammeln

In der Praxis kann die Vorrichtung ein Instrument zum Entfernen der Flüssigkeit umfassen, sodass die Flüssigkeit nach dem immobilisieren der Biomoleküle an der Oberfläche der magnetischen Partikel aus dem Behälter entfernt werden kann. Das Instrument zum Entfernen der Flüssigkeit kann insbesondere eine Pipette, ein Ventil, Druckluft oder eine anderes geeignetes Instrument sein.

Erfindungsgemäss wird weiter ein Verfahren zur reversiblen Immobilisierung von Biomolekülen vorgeschlagen. Das Verfahren umfasst dabei folgende Schritte. Zuerst werden magnetischen Partikeln und eine Flüssigkeit mit Biomolekülen in einem Behälter angeordnet. Dann werden die Biomoleküle an die magnetischen Partikel gebunden, insbesondere reversibles gebunden.

Die magnetischen Partikel mit den immobilisierten Biomolekülen werden im Behälter in einem durch Anordnung des Magneten erzeugten inhomogenen Magnetfeld fixiert, sodass sich die magnetischen Partikel strukturiert anordnen. Anschliessend wird die Flüssigkeit mit einem Instrument zum Entfernen einer Flüssigkeit entfernt, wobei die Flüssigkeit durch strukturierte Anordnung der magnetischen Partikel von den magnetischen Partikel abfliesst, sodass keine beziehungsweise nur wenig Flüssigkeitsrest im Behälter und auf den magnetischen Partikeln verblieben. Die an den magnetischen Partikeln gebundenen Biomoleküle können von der Oberfläche der magnetischen Partikel abgelöst werden und anschliessend weiter verwendet werden.

Das vorrangehend beschriebene Verfahren wird bevorzugt mit einer erfindungsgemässen Vorrichtung durchgeführt.

Erfindungsgemäss wird ausserdem ein Apparat zur automatisierten Verarbeitung von Biomolekülen umfassend eine erfindungsgemässe Vorrichtung vorgeschlagen. In dem Apparat zur automatisierten Verarbeitung von Biomolekülen kann zum Beispiel ein erfindungsgemässes Verfahren durchgeführt werden. Der Vorteil eines solchen Apparaten besteht darin, dass die Flüssigkeit mit Biomolekülen und die magnetischen Partikel durch geeignete Elemente in den Behälter zugeführt, sowie aus dem Behälter entfernt werden können. Ausserdem kann gegebenenfalls die Magnetposition am Behälter verändert werden, um zum Beispiel die magnetischen Partikel aus dem Behälter zu entfernen. Typischerweise werden bei Apparaten zur automatisierten Verarbeitung von Biomolekülen Multiwellplatten als Behälter verwendet.

Im Folgenden werden die Erfindung und der Stand der Technik anhand von Ausführungsbeispielen unter Bezugnahme auf die Zeichnungen näher erläutert.
- Fig. 1: eine schematische Darstellung einer Vorrichtung zur reversiblen Immobilisierung von Biomolekülen mit einer Multiwellplatte und einem magnetisch leitfähigen Modul
- Fig. 2: eine schematische Darstellung verschiedener Formen des Magneten und des magnetisch leitfähigen Moduls
- Fig. 3: eine schematische Darstellung eines weiteren Ausführungsbeispiels einer Vorrichtung zur reversiblen Immobilisierung von Biomolekülen
- Fig. 4: eine schematische Darstellung eines Magneten mit magnetisch leitfähigem Modul in Kronenform
- Fig. 5: eine schematische Darstellung des Standes der Technik im Vergleich zur Erfindung in der Draufsicht, sowie Ausführungen der Erfindung in der Seitenansicht
- Fig. 6: eine schematische Darstellung eines Ringmagneten mit magnetisch leitfähigem Modul als magnetisch Verstärkendes Modul und diamagnetisches Modul

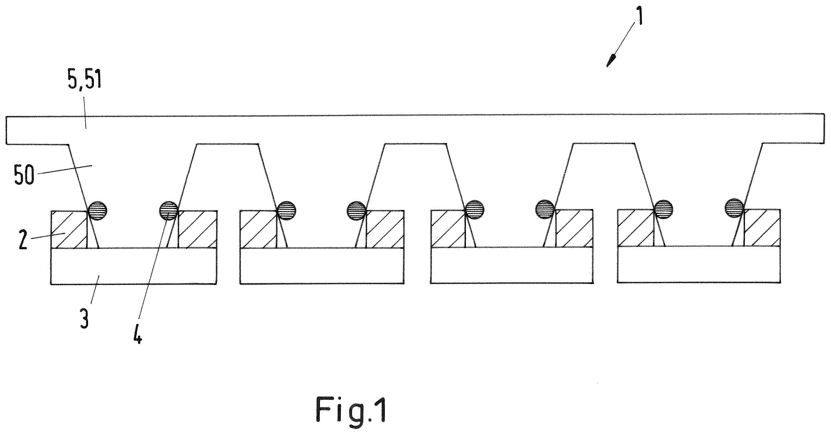

Figur 1 zeigt eine schematische Darstellung einer Vorrichtung 1 zur reversiblen Immobilisierung von Biomolekülen mit einer Multiwellplatte 51 und einem magnetisch leitfähigen Modul 2. Bei der gezeigten Vorrichtung 1 ist die Anordnung des Magneten 3 mit einem magnetisch leitfähigen Modul 2 ausgeführt. Das magnetisch leitfähige Modul 2 ist als Aufsatz auf dem Magneten 3 derart angeordnet, dass es sich zwischen Magneten 3 und Vertiefungen 50 der Multiwellplatte 51 befindet.

Durch die vorrangehend beschriebene Anordnung des Magneten 3 mit dem magnetisch leitfähigen Modul 2 ordnen sich die magnetischen Partikel 4 strukturiert im Behälter an. Im Betriebszustand kann eine Flüssigkeit, nach immobilisieren der Biomoleküle an der Oberfläche der magnetischen Partikel mit einem Instrument zum Entfernen einer Flüssigkeit (hier nicht gezeigt) entfernt werden und die Flüssigkeit einfach zwischen den strukturiert angeordneten magnetischen Partikeln abfliessen.

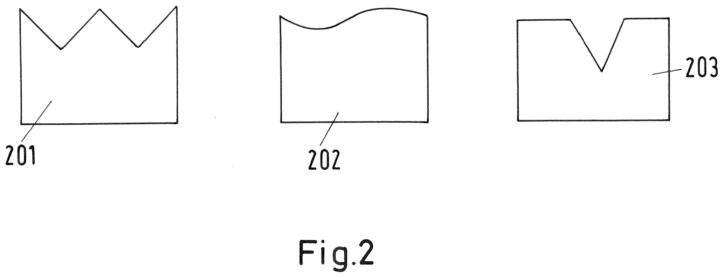

Figur 2 zeigt eine schematische Darstellung verschiedener Formen des Magneten 3 und des magnetisch leitfähigen Moduls 2. Der Magnet 3 kann zum Beispiel als kronenförmiger Magnet 201, als wellenförmiger Magnet 202 und als eingekerbter Magnet 203 ausgestaltet sein. Selbstverständlich kann auch das magnetische leitfähige Modul kronenförmig, wellenförmig oder mit Einkerbung ausgestaltet sein. Durch die Kronenform ordnen sich die magnetischen Partikeln in mehreren isolierten Inseln an. Die Anzahl der Inseln von magnetischen Partikeln entspricht der Anzahl der Zacken der Krone. Auch bei der Wellenform würden sich die magnetischen Partikeln so anordnen. Bei einer Einkerbung hingegen ordnen sich die magnetischen Partikel in zwei isolierten Inseln an.

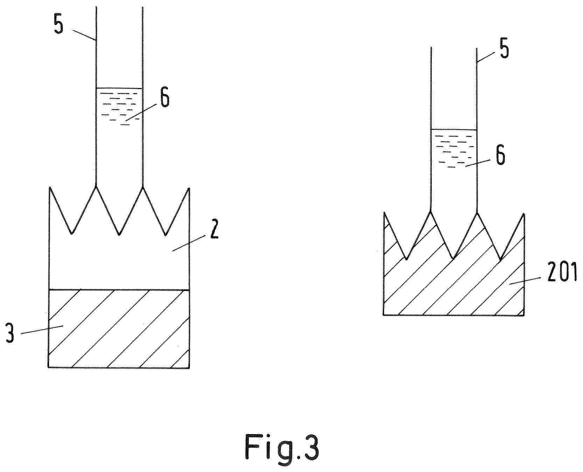

Figur 3 zeigt eine schematische Darstellung eines weiteren Ausführungsbeispiels einer Vorrichtung 1 zur reversiblen Immobilisierung von Biomolekülen. Es ist ein Behälter 5 gezeigt in welchen eine Flüssigkeit 6 mit Biomolekülen gefüllt ist.

Im Betriebszustand würden die Biomoleküle an der Oberfläche der magnetischen Partikel (hier nicht gezeigt) immobilisiert werden. Anschliessend würde die Flüssigkeit 6 aus dem Behälter entfernt werden.

Weiterhin ist in Figur 3 gezeigt dass ein Magnet 3 mit magnetisch leitfähigen Modul 2 am Behälter angeordnet sein kann. Das magnetisch leitfähige Modul 2 ist hierbei als kronenförmiger Aufsatz ausgeführt. Alternativ kann auch ein kronenförmiger Magnet 201 am Behälter angeordnet sein. Beide gezeigten Anordnungen weisen eine Form auf, welche an die Form des Behälters 5 angepasst ist, sodass der Behälter teilweise in den Magneten beziehungsweise das magnetisch leitfähige Modul eingeführt werden kann.

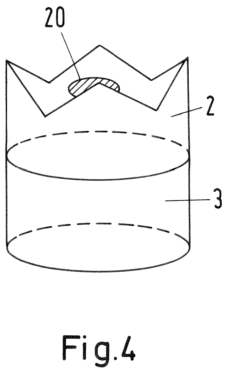

Figur 4 zeigt eine schematische Darstellung eines Magneten mit magnetisch leitfähigem Modul 2 in Kronenform. Das magnetisch leitfähige Modul 2 ist hierbei als Aufsatz für den Magneten 3 ausgestaltet. Das magnetisch leitfähige Modul 2 weist ein Loch 20, in welches ein Behälter zu Befestigung eingesteckt werden kann.

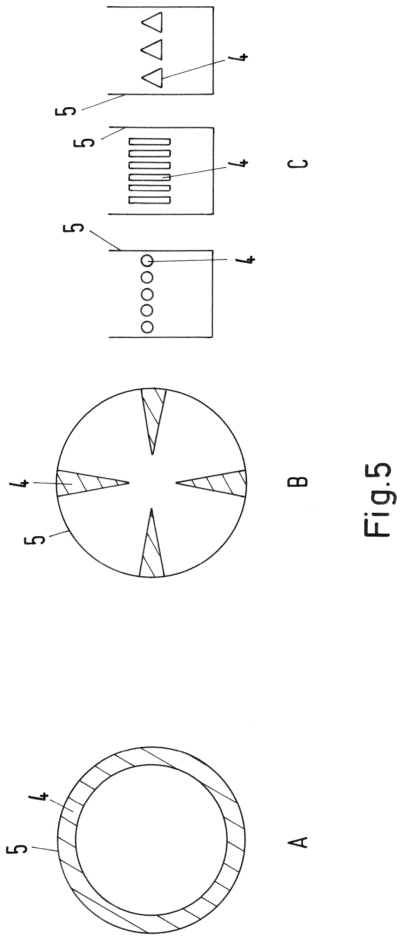

Figur 5 zeigt eine schematische Darstellung des Standes der Technik A im Vergleich zur Erfindung B in der Draufsicht auf den Behälter 5, sowie Ausführungen der Erfindung C in der Seitenansicht des Behälters 5.

Beim Stand der Technik A ordnen sich die magnetischen Partikel 4 durch das homogene Magnetfeld des Magneten in einem Ring am Rand des Behälters 5 an. Auf diesem Ring verbleibt die Flüssigkeit beim Entfernen, da sie nicht abfliessen kann.

Bei der Erfindung B ordnen sich die magnetischen Partikel 4 durch das inhomogene Magnetfeld des Magneten strukturiert an der Behälterwand an. Durch das hier gezeigt anordnen der magnetischen Partikel 4 in mehreren isolierten Inseln kann die Flüssigkeit einfach zwischen den magnetischen Partikeln 4 abfliessen.

In Teil C von Figur 5 sind drei Beispiele von Anordnungsmöglichkeiten der magnetischen Partikel 4 in einem erfindungsgemässen inhomogenen Magnetfeld an der Behälterwand des Behälters 5 gezeigt. Es sind eine Anordnung in mehreren rundlichen Inseln gezeigt, sowie eine rillenförmige und eine pyramidale Anordnung der magnetischen Partikel 4 gezeigt. All diese Anordnungen sind nur beispielhaft und nicht beschränkend. Es soll lediglich diverse Möglichkeiten aufgezeigt werden. In einem erfindungsgemässen inhomogenem Magnetfeld können sich magnetischen Partikel selbstverständlich in jeder beliebigen geeigneten Struktur anordnen, welche ein vereinfachtes Ablaufen der Flüssigkeit erlaubt.

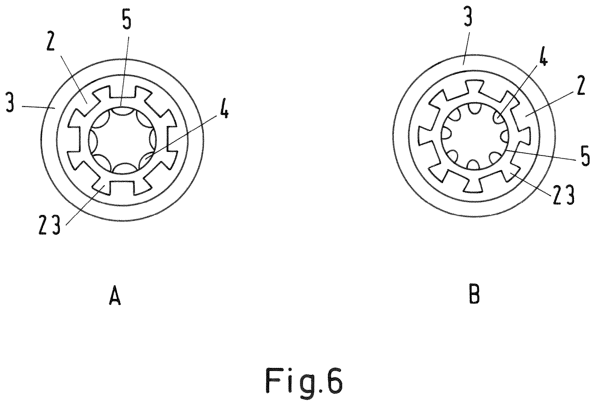

Figur 6 zeigt eine schematische Darstellung eines Ringmagneten 3 mit magnetisch leitfähigem Modul 2 als magnetisch verstärkendes Modul und diamagnetisches Modul.

In Teil A von Figur 6 ist das magnetisch leitfähige Modul 2 ein magnetisch verstärkendes Modul. Das magnetisch verstärkende Modul ist als Einsatz für einen Ringmagneten 3 ausgestaltet und zwischen Ringmagnet 3 und Behälter 5 angeordnet. Durch das magnetisch verstärkende Modul wird das Magnetfeld des Ringmagneten 3 in Bereichen ohne Lücke 23 mehr verstärkt und somit inhomogen. Die magnetischen Partikel 5 ordnen sich somit strukturiert an der Wand des Behälters 5 zwischen den Lücken 23 an.

In Teil B von Figur 6 ist das magnetisch leitfähige Modul 2 ein diamagnetisches Modul. Das diamagnetische Modul ist als Einsatz für einen Ringmagneten 3 ausgestaltet und zwischen Ringmagnet 3 und Behälter 5 angeordnet. Durch das diamagnetische Modul wird das Magnetfeld des Ringmagneten 3 in Bereichen ohne Lücke 23 stärker abgeschwächt und somit inhomogen. Die magnetischen Partikel 5 ordnen sich somit strukturiert an der Wand des Behälters 5 in den Lücken 23 an.

## Patentansprüche

1. Vorrichtung (1) zur reversiblen Immobilisierung von Biomolekülen mittels magnetischer Partikel (4), wobei die Vorrichtung (1) einen mit einer Flüssigkeit mit Biomolekülen befüllbaren Behälter (5, 51) und einen Magnet (3) umfasst, wobei der Magnet (3) derart am Behälter (5, 51) angeordnet ist, dass im Behälter (5, 51) anordnenbare magnetische Partikel (4) an welchen die Biomoleküle immobilisierbar sind, insbesondere reversibel immobilisierbar sind, im Behälter (5, 51) fixierbar sind, und
durch Anordnung des Magneten (3) ein, auf die im Behälter (5, 51) befindlichen magnetischen Partikel (4) wirkendes, inhomogenes Magnetfeld erzeugbar ist, sodass die magnetischen Partikel (4) durch Beeinflussung von dem inhomogenen Magnetfeld derart in mehreren isolierten Inseln an einer Behälterwand des Behälters (5,51) anordnenbar sind, dass die Flüssigkeit zwischen den isolierten Inseln von den magnetischen Partikeln (4) abfliessen kann,
**dadurch gekennzeichnet, dass**
der Magnet ein Loch zum Einstecken des Behälters (5) umfasst.

2. Vorrichtung (1) nach Anspruch 1, wobei die Anordnung des Magneten (3) derart ausgeführt ist, dass der Magnet (3) ein magnetisch leitfähiges Modul (2) umfasst, sodass durch das magnetisch leitfähige Modul (2) ein auf die im Behälter befindlichen magnetischen Partikel wirkendes, inhomogenes Magnetfeld erzeugbar ist.

3. Vorrichtung (1) nach Anspruch 2, wobei das magnetisch leitfähige Modul (2) als ein Bauteil am Magneten (3) angeordnet ist oder das magnetisch leitfähige Modul (2) als ein integriertes Element des Magneten (3) ausgeführt ist.

4. Vorrichtung (1) nach Anspruch 2 oder 3, wobei das magnetisch leitfähige Modul (2) ein magnetisch verstärkendes Modul (21) und/oder ein diamagnetisches Modul (22) ist.

5. Vorrichtung (1) nach einem der Ansprüche 1 bis 4, wobei die Form des Magneten (3) an die Form des Behälters (5, 51) angepasst ist, sodass der Behälter (5, 51) in das magnetisch leitfähige Modul (2) einführbar ist.

6. Vorrichtung (1) nach einem der Ansprüche 1 bis 5, wobei der Behälter (5) ein Multiwellplatte ist und die Multiwellplatte mehrere Vertiefungen aufweist.

7. Vorrichtung (1) nach Anspruch 6, wobei an mehreren Vertiefungen de Multiwellplatte ein Magnet (3) angeordnet ist.

8. Vorrichtung (1) nach Anspruch 1 oder 6, wobei die Anordnung des Magneten (3) derart ausgeführt ist, dass ein zweiter Magnet am Magneten (3) derart angeordnet ist, dass das erste Magnetfeld des Magneten (3) durch das zweite Magnetfeld des zweiten Magneten beeinflussbar ist, sodass ein, auf die im Behälter befindlichen magnetischen Partikel wirkendes, inhomogenes Magnetfeld erzeugbar ist

9. Vorrichtung (1) nach einem der Ansprüche 1 bis 7, wobei die Anordnung des Magneten (3) derart ausgeführt ist, dass der Magnet (3) eine Einkerbung aufweist, sodass durch die Einkerbung des Magneten (3) ein inhomogenes Magnetfeld erzeugbar ist.

10. Vorrichtung (1) nach einem der vorrangehenden Ansprüche, wobei der Magnet (3) ein Permanentmagnet und/oder ein Elektromagnet ist.

11. Vorrichtung (1) nach einem der vorrangehenden Ansprüche, wobei die Vorrichtung (1) ein Instrument (6) zum Entfernen der Flüssigkeit umfasst.

12. Verfahren zur reversiblen Immobilisierung von Biomolekülen mit einer Vorrichtung nach einem der vorangehenden Ansprüche,
**dadurch gekennzeichnet, dass**
das Verfahren folgende Schritte umfasst:
f) Anordnen von magnetischen Partikeln (4) und einer Flüssigkeit (6) mit Biomolekülen in einem Behälter (5, 51)
g) Binden, insbesondere reversibles binden, der Biomoleküle an die magnetischen Partikel (4)
h) Fixieren der magnetischen Partikel (4) im Behälter (5) in einem durch Anordnung des Magneten (3) erzeugten inhomogenen Magnetfeld, sodass sich die magnetischen Partikel (4) in mehreren isolierten Inseln an einer Behälterwand des Behälters (5,51) anordnen
i) Entfernen der Flüssigkeit mit einem Instrument zum Entfernen einer Flüssigkeit, wobei die Flüssigkeit durch die Anordnung in mehreren isolierten Inseln zwischen den isolierten Inseln von den magnetischen Partikel (4) abfliesst.
j) Lösen der Biomoleküle von den magnetischen Partikeln (4).

13. Apparat zur automatisierten Verarbeitung von Biomolekülen umfassend eine Vorrichtung (1) nach einem der Ansprüche 1 bis 11.

## Claims

1. A device (1) for the reversible immobilization of biomolecules by means of magnetic particles (4), the device (1) comprising a container (5, 51) which can be filled with a liquid containing biomolecules and a magnet (3), wherein the magnet (3) is arranged on the container (5, 51) in such a way that magnetic particles (4), which can be arranged in the container (5, 51), and to which the biomolecules can be immobilized, in particular can be reversibly immobilized, can be fixed in the container (5, 51), and that an inhomogeneous magnetic field acting on the magnetic particles (4) located in the container (5, 51) can be generated by the arrangement of the magnet (3), so that the magnetic particles (4) can be arranged in several isolated islands on a container wall of the container (5, 51) by the influence of the inhomogeneous magnetic field in such a way that the liquid between the isolated islands can flow off from the magnetic particles (4),
**characterized in that**
the magnet comprises a hole for inserting the container (5).

2. A device (1) according to claim 1, wherein the arrangement of the magnet (3) is performed in such a way that the magnet (3) comprises a magnetically conductive module (2), so that an inhomogeneous magnetic field acting on the magnetic particles located in the container can be generated by the magnetically conductive module (2).

3. A device (1) according to claim 2, wherein the magnetically conductive module (2) is arranged as a component on the magnet (3) or wherein the magnetically conductive module (2) is designed as an integrated element of the magnet (3).

4. A device (1) according to claim 2 or 3, wherein the magnetically conductive module (2) is a magnetically amplifying module (21) and / or a diamagnetic module (22).

5. A device (1) according to anyone of the claims 1 to 4, wherein the shape of the magnet (3) is adapted to the shape of the container (5, 51), so that the container (5, 51) can be inserted into the magnetically conductive module (2).

6. A device (1) according to anyone of the claims 1 to 5, wherein the container (5) is a multiwell plate, and the multiwell plate has a plurality of wells.

7. A device (1) according to claim 6, wherein a magnet (3) is arranged at a plurality of wells of the multiwell plate.

8. A device (1) according to claim 1 or 6, wherein the arrangement of the magnet (3) is performed in such a way that a second magnet is arranged on the magnet (3) in such a way that the first magnetic field of the magnet (3) can be influenced by the second magnetic field of the second magnet, so that an inhomogeneous magnetic field acting on the magnetic particles located in the container can be generated.

9. A device (1) according to anyone of the claims 1 to 7, wherein the arrangement of the magnet (3) is performed in such a way that the magnet (3) has a notch, so that an inhomogeneous magnetic field can be generated by the notch of the magnet (3).

10. A device (1) according to anyone of the preceding claims, wherein the magnet (3) is a permanent magnet and / or an electromagnet.

11. A device (1) according to anyone of the preceding claims, wherein the device (1) comprises an instrument (6) for removing the liquid.

12. A method for the reversible immobilization of biomolecules by means of a device according to anyone of the preceding claims,
**characterized in that**
the method comprises the following steps:
a) arranging magnetic particles (4) and a liquid (6) with biomolecules in a container (5, 51)
b) bonding, in particular reversible bonding, the biomolecules to the magnetic particles (4)
c) fixing the magnetic particles (4) in the container (5) in an inhomogeneous magnetic field generated by the arrangement of the magnet (3), so that the magnetic particles (4) arrange themselves in several isolated islands on a container wall of the container (5, 51)
d) removing the liquid with an instrument for removing a liquid, wherein the liquid between the isolated islands flows off from the magnetic particles (4) by the arrangement in several isolated islands
e) detaching the biomolecules from the magnetic particles (4).

13. An apparatus for the automated processing of biomolecules comprising a device (1) according to anyone of the claims 1 to 11.

## Revendications

1. Dispositif (1) pour l'immobilisation réversible de biomolécules au moyen de particules magnétiques (4), le dispositif (1) comprenant un récipient (5, 51) qui peut être rempli d'un liquide contenant des biomolécules et un aimant (3), dans lequel l'aimant (3) est disposé sur le récipient (5, 51) de telle sorte que des particules magnétiques (4), qui peuvent être disposées dans le récipient (5, 51) et auxquelles les biomolécules peuvent être immobilisées, en particulier peuvent être immobilisées de manière réversible, peuvent être fixées dans le récipient (5, 51), et qu'un champ magnétique inhomogène agissant sur les particules magnétiques (4) se trouvant dans le récipient (5, 51) peut être généré par la disposition de l'aimant (3), de sorte que les particules magnétiques (4) peuvent être disposées dans plusieurs îlots isolés sur une paroi de récipient du récipient (5, 51) sous l'influence du champ magnétique inhomogène de telle sorte que le liquide entre les îlots isolés peut s'écouler des particules magnétiques (4),
**caractérisé en ce que**
l'aimant comprend un trou pour insérer le récipient (5).

2. Dispositif (1) selon la revendication 1, dans lequel la disposition de l'aimant (3) est réalisée de telle sorte que l'aimant (3) comprend un module magnétiquement conducteur (2), de sorte qu'un champ magnétique inhomogène agissant sur les particules magnétiques se trouvant dans le récipient peut être généré par le module magnétiquement conducteur (2).

3. Dispositif (1) selon la revendication 2, dans lequel le module magnétiquement conducteur (2) est disposé en tant que composant sur l'aimant (3) ou dans lequel le module magnétiquement conducteur (2) est conçu en tant qu'élément intégré de l'aimant (3).

4. Dispositif (1) selon la revendication 2 ou 3, dans lequel le module magnétiquement conducteur (2) est un module d'amplification magnétique (21) et/ou un module diamagnétique (22).

5. Dispositif (1) selon l'une quelconque des revendications 1 à 4, dans lequel la forme de l'aimant (3) est adaptée à la forme du récipient (5, 51), de sorte que le récipient (5, 51) peut être inséré dans le module magnétiquement conducteur (2).

6. Dispositif (1) selon l'une quelconque des revendications 1 à 5, dans lequel le récipient (5) est une plaque à puits multiples, et la plaque à puits multiples a une pluralité de puits.

7. Dispositif (1) selon la revendication 6, dans lequel un aimant (3) est disposé sur une pluralité de puits de la plaque à puits multiples.

8. Dispositif (1) selon la revendication 1 ou 6, dans lequel la disposition de l'aimant (3) est réalisée de telle sorte qu'un deuxième aimant est disposé sur l'aimant (3) de telle sorte que le premier champ magnétique de l'aimant (3) peut être influencé par le deuxième champ magnétique du deuxième aimant, de sorte qu'un champ magnétique inhomogène agissant sur les particules magnétiques se trouvant dans le récipient peut être généré.

9. Dispositif (1) selon l'une quelconque des revendications 1 à 7, dans lequel la disposition de l'aimant (3) est réalisée de telle sorte que l'aimant (3) a une encoche, de sorte qu'un champ magnétique inhomogène peut être généré par l'encoche de l'aimant (3).

10. Dispositif (1) selon l'une quelconque des revendications précédentes, dans lequel l'aimant (3) est un aimant permanent et/ou un électroaimant.

11. Dispositif (1) selon l'une quelconque des revendications précédentes, dans lequel le dispositif (1) comprend un instrument (6) pour retirer le liquide.

12. Procédé pour l'immobilisation réversible de biomolécules au moyen d'un dispositif selon l'une quelconque des revendications précédentes,
**caractérisé en ce que**
le procédé comprend les étapes suivantes :
f) disposition de particules magnétiques (4) et d'un liquide (6) avec des biomolécules dans un récipient (5, 51)
g) liaison, en particulier liaison réversible, des biomolécules aux particules magnétiques (4)
h) fixation des particules magnétiques (4) dans le récipient (5) dans un champ magnétique inhomogène généré par la disposition de l'aimant (3) de sorte que les particules magnétiques (4) se disposent elles-mêmes dans plusieurs îlots isolés sur une paroi de récipient du récipient (5, 51)
i) retrait du liquide avec un instrument pour retirer un liquide, dans lequel le liquide entre les îlots isolés s'écoule des particules magnétiques (4) par la disposition dans plusieurs îlots isolés
j) séparation des biomolécules des particules magnétiques (4).

13. Appareil pour le traitement automatisé de biomolécules comprenant un dispositif (1) selon l'une quelconque des revendications 1 à 11.
